# EUROPEAN PATENT APPLICATION

(11) **EP 4 047 082 A1**
(43) Date of publication of application: **24.08.2022**
(21) Application number: 20875806.0
(22) Date of filing: 16.10.2020
(51) Int. Cl.: C12N 5/071, C12N 5/078, C12Q 1/02

(54) **METHOD FOR EVALUATING DRUG TOXICITY**

(30) Priority: 17.10.2019 JP 2019189838
(71) Applicant: Public University Corporation Yokohama City University, Yokohama-shi, Kanagawa 236-0027 (JP); Takeda Pharmaceutical Company Limited, Osaka-shi, Osaka 541-0045 (JP)
(72) Inventor: TAKEBE, Takanori, Tokyo 113-8510 (JP); SAIKI, Norikazu, Tokyo 113-8510 (JP)
(74) Representative: Jones, Nicholas Andrew
(86) International application number: PCT/JP2020/039023
(87) International publication number: WO 2021/075528

(57) **Abstract**

The present invention provides drug toxicity evaluation platforms (such as an evaluation method and a kit therefor) that enable detailed analysis of the possibility and the like of developing drug-induced damage (such as DILI) to the liver and other organs. The method for evaluating drug toxicity of the present invention includes: a step of adding a drug to a co-culture system of an organoid and blood cells; and a step of evaluating the toxicity of the drug to the organoid.

## Description

### Technical Field

The present invention relates to an evaluation system that enables prediction or the like of the possibility of developing a drug-induced disorder of the liver and other organs (e.g., drug-induced liver injury).

### Background Art

The drug-induced liver injury (DILI) is a side effect that may occur during the course of continuing drug therapy. The drug-induced liver injury is classified into an addictive type, an allergic idiosyncratic type, and a metabolic idiosyncratic type, according to the mechanism of development. The addictive type is a dose-dependent liver injury caused by the toxicity of the drug itself or the metabolites of the drug, which is induced by acetaminophen, methotrexate, or the like. The allergic idiosyncratic type is a non-dose-dependent liver injury caused by autoimmunity due to the acquisition of antigenicity against the drug itself or metabolites of the drug, which is induced by ticlopidine HCl, loxoprofen Na, phenytoin, carbamazepine, rifampicin, terbinafine HCl, or the like. The metabolic idiosyncratic type is a duration-dependent liver injury caused by an increase in hepatotoxic metabolites due to a genetic predisposition such as metabolic enzymes, which is induced by diclofenac Na, isoniazid, acarbose, or the like. It is important to predict the possibility of developing DILI for each patient before administering a drug, so as to select an appropriate drug, and there is a demand for establishing an evaluation system for that purpose.

Patent Literature 1 discloses a method for evaluating allergic drug-induced liver injury, comprising "a step of co-culturing immune cells derived from a subject with hepatocytes expressing a drug-metabolizing enzyme in a medium containing a drug to be tested in the state where they are separated by a membrane that permeates drugs and their metabolites but does not permeate cells" and "a step of analyzing the immune cells after the first step".

Further, Non Patent Literature 1 also discloses a DILI evaluation system, similar to Patent Literature 1, in which a hepatocyte line (HepG2) is co-cultured with a monocyte/macrophage cell line (THP-1), describing that there was a difference in reactivity of hepatocytes between a DILI drug (such as troglitazone, trovafloxacin, diclofenac, and ketoconazole) and a non-DILI drug (such as rosiglitazone, levofloxacin, acetyl salicylic acid, and fluconazole) depending on the presence or absence of an inflammatory factor such as LPS and TNF.

### Citation List

### Patent Literature

Patent Literature 1: JP 2016-202032 A (JP 6516255 B)

### Non Patent Literature

Non Patent Literature 1: A. Granitzny et al. Toxicology Reports 4 (2017) 89-103

### Summary of Invention

### Technical Problem

As evaluation systems for DILI, those mentioned in the Background Art have been proposed, but there has been still room for improvement to enable a detailed analysis for each patient, for example, by proposing an alternative drug, while avoiding drugs that may develop DILI.

It is an object of the present invention to provide drug toxicity evaluation platforms (such as an evaluation method and a kit therefor) that enable detailed analysis of the possibility and the like of developing drug-induced damage (such as DILI) to the liver and other organs.

### Solution to Problem

The inventors have found that the use of a liver organoid co-cultured with blood cells (e.g., immunocompetent cells such as monocytes and macrophages) as a DILI evaluation system accurately reflects the possibility of developing drug-induced DILI. Specifically, it turned out that, when the liver organoid was produced using cells derived from a DILI patient, the toxicity to the liver organoid was observed even with addition of a small amount of DILI drug (e.g., ampicillin as an antibacterial drug), and thus the possibility of developing drug-induced DILI can be evaluated well. Further, it turned out that there were cases that DILI was not observed even if the liver organoid was produced using cells derived from a DILI patient, in the case of adding another drug (e.g., amoxicillin and cephalexin) used for the same purpose as that of the DILI drug, and it can be determined that such a drug may be an alternative drug to the DILI patient.

That is, the present invention provides [1] to [7] below in order to achieve the aforementioned object.
[1] A method for evaluating drug toxicity, comprising:
   a step of adding a drug to a co-culture system of an organoid and blood cells; and
   a step of evaluating the toxicity of the drug to the organoid.
[2] The method for evaluating drug toxicity according to item 1, wherein the blood cells are immunocompetent cells.
[3] A method for presuming a biomarker, comprising a step of performing the method for evaluating drug toxicity according to item 1 or 2 for multiple specimens of the organoid and comparing substances in culture supernatants between specimens having drug toxicity and specimens without drug toxicity to presume a substance to serve as a biomarker.
[4] A method for screening a drug, comprising a step of performing the method for evaluating drug toxicity according to item 1 or 2 for multiple drugs using an organoid derived from a patient having developed drug toxicity to select a drug with low drug toxicity.
[5] A kit for evaluating drug toxicity, comprising an organoid and blood cells.
[6] The kit according to item 5, further comprising a dead cell detection reagent.
[7] A culture comprising an organoid, blood cells and a drug.

### Advantageous Effects of Invention

Use of such a drug toxicity evaluation platform (such as an evaluation method and a kit therefor) by the present invention enables the toxicity to the patient's organs to be accurately determined for each drug to select a less toxic drug, and a mechanism of action (such as biomarkers) of mitigating the toxicity of the drug and a new drug with lower toxicity than before to be found.

The reasons why the method for evaluating drug toxicity of the present invention using an organoid together with blood cells is excellent are presumed to be, for example, that the three dimensional organ structure including vascular endothelial cells is closer to that in vivo, so that the interaction between drug toxicity and blood cells can be reproduced with high accuracy, and the activity of metabolic enzymes important for evaluation is high due to factors such as cell maturation caused by the interaction between the cells. Further, since blood cells, preferably immunocompetent cells are used in the method for evaluating drug toxicity of the present invention, the reproducibility of toxicity in vitro is improved, particularly when such cells (immune system) are involved in the drug toxicity, so that the toxicity can be reproduced more accurately (with high S/N ratio), which has been difficult with conventional evaluation methods.

### Description of Embodiments

### -Method for evaluating drug toxicity-

The method for evaluating drug toxicity of the present invention comprises at least the following steps and may further include other steps, as required.
Step 1 (drug adding step): a step of adding a drug to a co-culture system of an organoid and blood cells.
Step 2 (toxicity evaluating step): a step of evaluating the toxicity of the drug to the organoid.

### <Organoid>

The "organoid" used in the present invention includes organ or tissue organoids (collectively referred to as "organ/tissue organoids" in this description), cancer organoids, and the like. The organoid includes not only organoids in the structurally mature stage such as of organs, tissues, and cancers, but also simple cell aggregates (spheroids).

The organoid may be derived from humans or derived from animals other than humans, for example, mammals such as mice, rats, dogs, pigs, and monkeys. That is, predetermined cells (see the following description for details) used for fabricating organoid may be cells derived from humans or cells derived from animals other than humans. For detecting drugs that cause drug addiction, which is difficult to find in conventional animal experiments or human cell tests in the development of human medicaments, the organoid is preferably derived from a human. Further, predetermined cells used for fabricating organoid may be primary cultured cells or subcultured cells (established cell strain).

In one preferable embodiment of the present invention, the organoid is derived from a patient (human) having developed drug toxicity. In this embodiment, the predetermined cells used for fabricating organoid are, for example, cells collected from a patient having developed drug toxicity (primary cultured cells) or iPS cells fabricated using the cells, or their subcultured cells (established cell strain). For example, iPS cell line fabricated using cells collected from a patient having developed drug toxicity is preferable for fabricating an organoid, that is, inducing differentiation into predetermined various cells to serve as raw materials for the organoid, in order to standardize the method for evaluating drug toxicity of the present invention.

An "organ/tissue organoid" is an artificially created organism (three-dimensional structure) similar to an organ or a tissue. Organ organoids of various types, such as liver, pancreas, kidney, heart, lung, spleen, esophagus, stomach, thyroid gland, parathyroid gland, thymus, reproductive gland, brain, spinal cord, skin, and inner ear have been already publicly known (e.g., see https://www.nejm.org/doi/pdf/10.1056/NEJMra1806175, https://www.nature.com/articles/s41568-018-0007-6, and http://www.amsbio.com/brochures/organoid-culture-handbook.pdf). The "organ/tissue organoid" also includes "organ buds" (e.g., liver buds and pancreatic buds), which are early-stage structures that eventually lead to organs with complex structures.

The method for fabricating an organ/tissue organoid (organ bud) is publicly known, and the fabrication method by which the organ/tissue organoid used in the present invention is obtained is not particularly limited. As a preferable example of the method for fabricating the organ/tissue organoid (organ bud), a liver bud can be fabricated by co-culturing cells constituting an organ or a tissue, mesenchymal cells, and vascular endothelial cells, as described in WO2015/129822.

The organ/tissue organoid (organ bud) to be used can be selected according to the purpose of the method for evaluating drug toxicity. For example, in the case of implementing the method for evaluating drug toxicity for hepatotoxicity, a liver organoid, which is an organ that actually induces a disorder (such as hepatocyte injury), may be used, or an organoid of the skin, the inner ear, and the like, which are organs that tend to develop allergic reactions in the case of allergic idiosyncratic drug-induced liver injury may be used.

Typical examples of the organ organoid in the present invention include "liver organoid". The liver organoid is preferably a "liver bud". The method for fabricating the liver organoid (liver bud) is publicly known, and a preferable example thereof is a method of co-culturing hepatic endoderm cells, mesenchymal cells, and vascular endothelial cells according to the method for fabricating organ organoids (organ buds) described in WO2015/129822.

A "cancer organoid" is a cell aggregate composed of cancer cells and other cells, which reproduces a cancer microenvironment. The method for fabricating a cancer organoid is publicly known, and a cancer organoid can be fabricated by co-culturing cancer cells, mesenchymal cells, and vascular endothelial cells, for example, as described in JP 2018-110575 A.

The cancer cells may be an existing cancer cell line or a primary cancer cell line established using a cancer tissue separated from the primary tumor of a human cancer. The type of the cancer is not particularly limited and may be liver cancer, renal cancer, malignant brain tumor, pancreatic cancer, gastric cancer, lung cancer, or the like, for example.

In the case of using a cancer organoid, it is possible to evaluate drug toxicity that worsens the symptoms of cancer or adversely affects other organs and even presume a biomarker involved in the drug toxicity, for example, due to an unusual metabolite occurring in cancer cells (cancerized tissue) to which a drug has been administered according to a typical embodiment of the present invention. Meanwhile, in the case of using a cancer organoid, the method for evaluating drug toxicity of the present invention can be changed to a method for evaluating a drug from another viewpoint other than toxicity, for example, a "method for evaluating drug resistance". It is said that the tumor microenvironment constructed by the interaction between cancer cells and various cells present in the periphery of the cancer cells (mesenchymal cells such as tumor-related fibroblasts and vascular endothelial cells, and inflammatory cells such as macrophages) plays an important role in the treatment resistance (such as drug resistance, radiation sensitivity, immunotherapy sensitivity, and nutrition therapy sensitivity) of cancers (such as pancreatic cancer). Accordingly, it is also possible to perform a method for evaluating drug sensitivity including a step of adding a drug to the co-culture system of the cancer organoid and the blood cells (such as immunocompetent cells) and a step of evaluating the drug sensitivity of the cancer organoid to the drug.

The "cells constituting an organ or a tissue" used for fabricating the organoid (organ/tissue organoid) includes (I) parenchymal cells constituting an organ or a tissue and (II) non-parenchymal cells constituting an organ or a tissue. Further, the parenchymal cells (I) and the non-parenchymal cells (II) each include (i) differentiated, mature, or terminally differentiated cells with a predetermined functionality as parenchymal cells or non-parenchymal cells (simply referred to as "differentiated cells" in this description), and (ii) cells capable of differentiating into parenchymal cells or non-parenchymal cells, or destined for differentiation (committed) but undifferentiated, or at the stage of stem cells or progenitor cells which do not sufficiently have a predetermined functionality as parenchymal cells or non-parenchymal cells (simply referred to as "undifferentiated cells" in this description).

As the cells constituting an organ or a tissue, at least one selected from the group consisting of differentiated cells of parenchymal cells, undifferentiated cells of parenchymal cells, differentiated cells of non-parenchymal cells, and undifferentiated cells of non-parenchymal cells, preferably a combination of two or more cells capable of forming an "organ/tissue organoid" (e.g., organ bud) can be used.

Examples of the "parenchymal cells" constituting an organ or a tissue include hepatocytes, pancreatic endocrine cells (e.g., α cells, β cells, δ cells, ε cells, and PP cells) and pancreatic duct epithelial cells, renal tubular epithelial cells and glomerular epithelial cells, pulmonary alveolar epithelial cells, cardiac cardiomyocytes, intestinal epithelial cells, cerebral nerve cells and glial cells, and spinal nerve cells and Schwann cells.

Examples of the "non-parenchymal cells" constituting an organ or a tissue include hepatic sinusoidal endothelial cells, hepatic stellate cells and Kupffer cells, pancreatic stellate cells and pancreatic microvascular endothelial cells, renal glomerular endothelial cells, pulmonary artery endothelial cells and lung fibroblasts, cardiac microvascular endothelial cells, aortic endothelial cells, coronary endothelial cells and cardiac fibroblasts, intestinal microvascular endothelial cells, cerebral microvascular endothelial cells, vascular pericytes, choroid plexus endothelial cells and cerebrovascular adventitial fibroblasts.

Examples of the "undifferentiated organ cells" capable of differentiating into the parenchymal cells or non-parenchymal cells constituting an organ or a tissue include cells capable of differentiating into ectodermal organs such as brain, spinal cord, adrenal medulla, epidermis, hair/nail/skin glands, sensory organs, peripheral nerves and lens; cells capable of differentiating into mesodermal organs such as kidney, ureter, heart, blood, reproductive glands, adrenal cortex, muscles, skeleton, dermis, connective tissue and mesothelium; and cells capable of differentiating into endodermal organs such as liver, pancreas, intestine, lungs, thyroid glands, parathyroid glands and urinary tract.

The "hepatocytes" used for fabricating a liver organoid (liver bud) refers to hepatic parenchymal cells and includes both concepts of differentiated hepatocytes (differentiated hepatocytes) and cells destined to differentiate into hepatocytes but have not yet differentiated into hepatocytes (undifferentiated hepatocytes), so-called hepatic progenitor cells (e.g., hepatic endoderm cells). The differentiated hepatocytes may be cells collected from a living body (isolated from the liver in vivo) or may be cells obtained by differentiating pluripotent stem cells such as ES cells and iPS cells, hepatic progenitor cells, or other cells having the ability to differentiate into hepatocytes. The undifferentiated hepatocytes may be collected from a living body or may be cells obtained by differentiating pluripotent stem cells such as ES cells and iPS cells, other stem cells, or progenitor cells. The cells capable of differentiating into hepatocytes can be fabricated, for example, according to K. Si-Taiyeb, et al. Hepatology, 51 (1): 297-305(2010), or T. Touboul, et al. Hepatology. 51 (5): 1754-65 (2010). The method for differentiating pluripotent stem cells such as ES cells and iPS cells, hepatic progenitor cells, or other cells having the ability to differentiate into hepatocytes is publicly known, and iPS cells can be differentiated, for example, according to the method described in Hepatology, 2010; 51 (1): 297-305, Cell Rep. 2017; 21 (10): 2661-2670, or the like. For both of the cell population collected from a living body and the cell population fabricated by inducing differentiation of ES cells, iPS cells, or the like (particularly, for the latter), a cell population with high purity of differentiated hepatocytes or a cell population with high purity of undifferentiated hepatocytes may be used, or a cell mixture containing differentiated hepatocytes and undifferentiated hepatocytes at any ratio may be used.

Whether or not a cell is a differentiated hepatocyte can be determined by determining whether or not the expression of one or more of mature hepatocyte markers, for example, asialoglycoprotein receptor 1 (ASGR1), α fetoprotein (AFP) as an immature hepatocyte marker (initial liver differentiation marker), albumin (ALB) as an initial liver differentiation marker, retinol-binding protein (RBP4), transthyretin (TTR), glucose-6-phosphatase (G6PC), and the like, are positive. Meanwhile, whether or not a cell is an undifferentiated hepatocyte can be determined by determining whether or not the expression of one or more cell markers such as HHEX, SOX2, HNF4α, AFP, and ALB are positive (for ALB, whether or not the expression thereof is weakly positive).

The "vascular endothelial cells" used for fabricating an organoid (such as an organ/tissue organoid and a cancer organoid) includes both concepts of hemogenic endothelial cells (HECs) and non-hemogenic endothelial cells (non-HECs). The HECs are vascular endothelial cells capable of producing hematopoietic stem cells (having hematopoietic ability), which are also called blood cell-producing vascular endothelial cells. Meanwhile, the non-HECs are vascular endothelial cells that do not have such hematopoietic ability.

The vascular endothelial cells may be a cell population with high purity of vascular endothelial cells collected from a living body (e.g., microvessel endothelial cells (MVECs), liver sinusoidal endothelial cells (LSECs), and umbilical-vein endothelial cells (UVECs), and the like) or may be a cell population with high purity of vascular endothelial cells obtained by differentiating pluripotent stem cells such as ES cells and iPS cells or other cells having the ability to differentiate into vascular endothelial cells.

The "mesenchymal cells" used for fabricating an organoid (such as an organ/tissue organoid and a cancer organoid) refers to connective tissue cells that exist mainly in a connective tissue derived from the mesoderm and form a support structure of cells that function in the tissue, and includes both concepts of differentiated cells (differentiated mesenchymal cells) and cells destined to differentiate into mesenchymal cells but have not yet differentiated into mesenchymal cells (undifferentiated mesenchymal cells), so-called mesenchymal stem cells. However, the "vascular endothelial cells" are one of cells differentiating from undifferentiated mesenchymal cells, but are excluded from the definition of "mesenchymal cells" in this description.

Whether a cell is an undifferentiated mesenchymal cell or a differentiated mesenchymal cell can be determined, for example, by determining whether or not one or more of Stro-1, CD29, CD44, CD73, CD90, CD105, CD133, CD271, Nestin, and the like, as undifferentiated mesenchymal cell markers are positive (it is determined to be an undifferentiated mesenchymal cell when they are positive, whereas it is determined to be a differentiated mesenchymal cell when they are negative).

The mesenchymal cells may further express a specific cell marker to a specific organ (tissue), according to the organ organoids intended in the present invention or the "cells constituting an organ or a tissue" to be used in combination. Examples of the cell marker include FOXF1, COL4A, and ALCAM, which are cell markers for septum transversum mesenchyme (STM).

### <Blood cells>

The "blood cells" used in the present invention include erythrocytes, leukocytes (mononuclear cells and granulocytes), and platelets. The "mononuclear cells" include lymphocytes and monocytes. The "lymphocytes" include NK cells, T cells (αβ T cells, γδ T cells, CD8⁺ T cells, CD4⁺ T cells, tumor infiltrating T cells, memory T cells, naive T cells, and NK T cells), and B cells. The "granulocytes" include neutrophils, eosinophils, and basophils.

The blood cells in the present invention are preferably immunocompetent cells. The "immunocompetent cells" are almost equivalent to the "mononuclear cells" and include T cells and B cells, which are responsible for acquired immunity, and neutrophils, NK cells, monocytes, macrophages, and dendritic cells, which are responsible for natural immunity. For example, T cells, neutrophils, NK cells, macrophages, and the like are preferable as blood cells (immunocompetent cells) to be co-cultured with the organoid in the drug adding step.

The blood cells, preferably, immunocompetent cells may be cells collected from a living body (e.g., mononuclear cells isolated from peripheral blood) or may be cells obtained by differentiating pluripotent stem cells such as ES cells and iPS cells or other cells having the ability to differentiate into blood cells. Further, the blood cells may be primary cultured cells or may be subcultured cells (established cell strain).

The blood cells may be derived from humans or derived from animals other than humans, for example, mammals such as mice, rats, dogs, pigs, and monkeys. For detecting drugs that cause drug addiction, which is difficult to find in conventional animal experiments or human cell tests in the development of human medicaments, the blood cells are preferably derived from a human. The animal species for blood cells is generally the same as the animal species for organoids (predetermined cells used for fabrication thereof).

In one preferable embodiment of the present invention, the blood cells are derived from a patient (human) having developed drug toxicity. That is, in one preferable embodiment of the present invention, the predetermined cells used for fabricating organoid are, for example, cells collected from a patient having developed drug toxicity (primary cultured cells) or iPS cells fabricated using the cells, or their subcultured cells (established cell strain).

### <Drug>

The "drug" used in the present invention is not particularly limited as long as it is a drug applied to organs, tissues, and the like (including cancerized ones) for which organoids are fabricated, and various drugs can be used according to the purpose of evaluating toxicity.

The drug-induced "toxicity" includes not only toxicity to organs such as liver as exemplified below but also "drug eruptions" (particularly allergic drug eruptions). The drug eruptions include toxic epidermal necrolysis, Stevens-Johnson syndrome, and virus-related drug-induced hypersensitivity syndrome.

The drug toxicity in the present invention is not limited to hepatotoxicity and may be, for example, toxicity to organs or tissues other than the liver, such as cardiotoxicity and blood/bone marrow toxicity. The method for evaluating drug toxicity of the present invention can be performed using an organoid of an organ or a tissue that may possibly develop drug toxicity (or those to be examined whether or not there is a possibility).

In one embodiment of the present invention, the drug is a drug to be evaluated for hepatotoxicity to the administration subject. Liver injury due to hepatotoxicity is generally classified into hepatocellular injury type, cholestasis type, and mixed type of these, or fulminant hepatitis.

The pathogenic mechanism of hepatotoxicity (drug-induced liver injury) is classified into addictive type and idiosyncratic type, and the idiosyncratic type may be further classified into metabolic idiosyncratic type and allergic idiosyncratic type. There are many reports of drugs related to each mechanism of action. For example, the "Drug Induced Liver Injury Rank (DILIrank) Dataset" of the FDA (Food and Drug Administration) publishes drugs related to drug-induced liver injury together with their severity classes. Also in the method for evaluating drug toxicity of the present invention, various drugs for which drug toxicity has been reported, or various drugs to serve as their alternatives (candidates) can be used. Even the same drug may be found to be involved in multiple mechanisms of action (e.g., both the allergic idiosyncratic type and the metabolic idiosyncratic type).

Drugs that may possibly develop addictive drug-induced liver injury themselves or metabolites thereof are hepatotoxic, and dose-dependent liver injury occurs in almost all subjects (humans). Although the addictive drug is relatively easy to reproduce in animal experiments and the like, it can also be used as a drug in the method for evaluating drug toxicity of the present invention. Examples of the addictive drug include acetaminophen (aspirin) and methotrexate.

Meanwhile, the drug that may possibly develop idiosyncratic drug-induced liver injury is relatively difficult to reproduce in poisonous experiments or the like and thus is preferable as a drug in the method for evaluating drug toxicity of the present invention. In the case of the allergic idiosyncratic type, it is thought that the drug itself or its reactive intermediate metabolite serves as a hapten and binds to various components of hepatocytes to acquire antigenicity, thereby causing an allergic reaction, which is developed often 1 to 8 weeks after taking the drug. Examples of the allergic idiosyncratic drug include ticlopidine HCl, loxoprofen Na, phenytoin, carbamazepine, rifampicin, and terbinafine HCl. Further, in the case of the metabolic idiosyncratic type, it is thought that the development is due to individual differences in metabolic enzyme activity in the liver, or the like, and is caused by long-term drug administration for 1 week (particularly after 8 weeks) to 1 year or longer. Examples of the metabolic idiosyncratic drug include acarbose, amiodarone, isoniazid, itraconazole, oral contraceptives, zafirlukast, diclofenac sodium, disulfiram, tamoxifen, anabolic steroids, dantrolene sodium, tegafur-uracil, terbinafine hydrochloride, troglitazone (sold to the public cancellation), sodium valproate, hydralazine hydrochloride, fluconazole, flutamide, pemoline, labetalol hydrochloride.

In recent years, the drug-induced liver injury may be classified into direct hepatotoxicity, idiosyncratic hepatotoxicity, and indirect hepatotoxicity (N Engl J Med 2019; 381:264-273). Examples of the "direct hepatotoxic" drug include high doses of acetaminophen, niacin, aspirin (acetyl salicylic acid), cocaine, IV amiodarone, IV methotrexate, and drugs used in cancer chemotherapy. Examples of the "idiosyncratic hepatotoxic" drug include amoxicillin/clavulanic acid, cephalosporin, isoniazid, nitrofurantoin, minocycline, fluoroquinolones, macrolides, and antibodies. Examples of the "indirect hepatotoxic" drug include anti-tumor agents, glucocorticoids, monoclonal antibodies (those against TNF, CD20, checkpoint proteins, and the like, such as anti-CTLA-4 antibody, anti-PD-1 antibody, and anti-PD-Ll antibody), and protein kinase inhibitors.

Examples of the more specific embodiment of the present invention include a method for evaluating hepatotoxicity using a chemotherapy drug (including an antifungal agent) or an antibacterial drug. Liver injuries such as hepatocellular injury type, mixed type, cholestasis type, and fulminant hepatitis have been reported for the chemotherapy drug, such as rifampicin, isoniazid (isonicotinic acid hydrazide: INH), salazosulfapyridine (sulfasalazine), ofloxacin, levofloxacin, norfloxacin, ciprofloxacin hydrochloride, sulfamethoxazole-trimethoprim, and griseofulvin. Further, liver injuries such as hepatitis type, mixed type, cholestasis type, and fulminant hepatitis have been reported for the antibacterial drug, such as cephems (such as cefotiam, cefaclor, cefazolin sodium, cefmethazole sodium, and cephalexin), carbapenems (such as imipenem-cilastatin sodium), penicillins (such as piperacillin sodium, ampicillin, amoxicillin, sulbactam sodium-ampicillin sodium combination drug, oxypenicillin, potassium clavulanate-amoxicillin combination drug, and tazobactam sodium-piperacillin sodium combination drug), macrolides (such as erythromycin estolate, roxithromycin, clarithromycin, and azithromycin), tetracyclines (such as tetracycline hydrochloride), minocycline hydrochloride, fosfomycin, cefteram pivoxil, cefpodoxime proxetil, flomoxef sodium, terbinafine hydrochloride, pyrazinamide, fluconazole, and itraconazole.

Other than the chemotherapy drug and the antibacterial drug, the following examples can be mentioned as drugs that may possibly develop drug-induced liver injury:
antipyretic anti-inflammatory analgesics such as diclofenac sodium, acetaminophen, loxoprofen sodium, acetyl salicylic acid, mefenamic acid, ibuprofen, indomethacin, pranoprofen, and sulindac;
psychiatric/neurological drugs such as phenytoin, carbamazepine, sodium valproate, chlorpromazine hydrochloride, haloperidol, dantrolene sodium, halothane, phenytoin (diphenyl hydantoin), and pemoline;
cardiovascular preparations (including anticoagulants) such as aprindine hydrochloride, ajmaline, trapidil, nifedipine, nicardipine hydrochloride, methyldopa, amiodarone, ticlopidine hydrochloride, hydrazine hydrochloride, and labetalol hydrochloride;
digestive drugs such as tiopronin, famotidine, lansoprazole, cimetidine, sulpiride, omeprazole, and ranitidine hydrochloride;
anticancer agents such as tegafur-uracil combination drug, cyclophosphamide, 6-mercaptopurine, cyclophosphamide, tamoxifen, flutamide, and methotrexate;
Chinese herbal medicine such as Shosaikoto, Saireito, and Kakkonto;
drugs for metabolic diseases (drugs for diabetes/hyperlipidemia) such as troglitazone, acarbose, voglibose, glibenclamide, and epalrestat;
and other drugs such as drugs for gout/hyperuricemia, respiratory drugs (e.g., zafirlukast), immunosuppressive drugs (e.g., azathioprine), urinary/genitourinary drugs, bone metabolism improving drugs, hormonal drugs (e.g., medical combination of estrogen preparation and progesterone preparation, and anabolic steroids), anti-allergic drugs, vitamin drugs, non-prescription drugs (e.g., vitamin A (retinol palmitate), acetaldehyde inhibitors (anti-alcohol therapy drugs such as disulfiram), and antithyroid drugs (e.g., propylthiouracil).

In one embodiment of the present invention, the drug is to be evaluated for cardiotoxicity to the administration subject. The cardiotoxicity is known to manifest, for example, as Torsade de Pointes (TdP) that is fatal arrhythmia, which causes a sudden cardiac death. Conventionally, the possibility of drug-induced TdP (TdP risk) has been evaluated based on the presence or absence of myocardial QT prolongation (prolongation of action potential duration), which is the previous stage, the presence or absence of hERG channel inhibitory effect, or the like. In the method for evaluating drug toxicity of the present invention, whether or not a drug has cardiotoxicity (TdP risk) can be evaluated, for example, by determining whether or not the waveform of the extracellular potential generated when the drug is applied to a cardiac organoid meets a predetermined standard or another technique. Various drugs that may possibly develop cardiotoxicity are publicly known and can be used in the present invention. Examples of the drugs having cardiotoxicity include anti-tumor drugs such as anthracyclines, cyclophosphamide, 5-fluorouracil, and taxanes; monoclonal antibodies such as trastuzumab, bevacizumab, and nivolumab; tyrosine kinase inhibitors such as sunitinib and nilotinib; antiretroviral drugs such as zidovudine; and anti-diabetic drugs such as rosiglitazone.

In one embodiment of the present invention, the drug is to be evaluated for blood/bone marrow toxicity to the administration subject. Examples of the blood/bone marrow toxicity induced by the drug include hypogloburia, leukopenia (agranulocytosis), thrombocytopenia, abnormal coagulation, bone marrow overgrowth, leukemia, and pancytopenia with hematopoiesis in three systems (erythrocyte system, granulocyte system, and megakaryocyte system). The blood/bone marrow toxicity is caused by hematopoietic disorders in hematopoietic organs (mainly bone marrow) and peripheral hematocrasia. The hematopoietic disorders are caused by the drug acting on myeloid stem cells or hematopoietic progenitor cells to suppress differentiation and growth. The hematocrasia is often caused by an immunological mechanism and is classified into drug adsorption type, immune complex type, and autoimmune type. Various drugs that may possibly develop blood/bone marrow toxicity are publicly known and can be used in the present invention. Irinotecan, methotrexate, and the like are typical drugs that cause bone marrow (and associated blood cells) toxicity. Examples of a drug that causes granulocytosis resulting in a decrease in neutrophils (drug-induced agranulocytosis) include antipyretic analgesics (non-steroidal anti-inflammatory drugs) such as aspirin, sulpyrine, diclofenac, and indomethacin; antipsychotics (antidepressants) such as chlorpromazine, levomepromazine, chlordiazepoxide, meprobamate, and clozapine; antithyroid hormonal drugs such as methylthio uracil, propylthiouracil, and thiamazole; diuretics such as chlorthalidone, chlorothiazide, and etacrynic acid; anticonvulsants (antiepileptic drugs) such as phenytoin, trimethadione, and carbamazepine; oral hypoglycemic drugs such as chlorpropamide and tolbutamide; antibiotics/antibacterial drugs such as chloramphenicol, penicillin, thiamphenicol, streptomycin, and trimethoprim sulfamethoxazole; anti-tuberculosis drugs such as para-aminosalicylic acid (PAS) and isoniazid (INH); antihistamines such as cyproheptadine and chlorpheniramine; antirheumatic drugs such as phenylbutazone, indomethacin, and gold compounds; antiarrhythmic drugs such as procainamide, ajmaline, and quinidine; antiplatelet drugs such as ticlopidine; peptic ulcer agents such as famotidine and lansoprazole; anticancer agents, penicillamine, and sulfonamides (sulfa drugs).

The "drugs" are not limited to low-molecular weight medicaments, as exemplified above, and may be various drugs other than the low-molecular weight medicaments, which can be used as active components of pharmaceutical products, such as antibody medicaments, peptide medicaments, and nucleic acid medicaments.

The "drugs" are not limited to drugs that are actually commercially available as pharmaceutical products and may be drugs used in clinical or non-clinical studies or drugs under development at the previous stage (such as candidate compounds for active components of pharmaceutical products).

### <Drug adding step>

The drug adding step that is the first step in the method for evaluating drug toxicity of the present invention is a step of adding a drug to a co-culture system of an organoid and blood cells.

In the drug adding step, an appropriate medium may be selected as the medium to culture the organoid according to the types of the organoid. Mediums for organoids are publicly known, and a medium mixed with the medium used for culturing the predetermined cells for fabricating each organoid, such as a mixed medium with a medium for cells constituting an organ or a tissue, a medium for mesenchymal cells, and a medium for vascular endothelial cells (the mediums may be common) can be generally used as the medium for organoids.

The drug can be added to the medium for organoids at a desired concentration suitable for evaluating drug toxicity. The concentration of the drug to be added can be appropriately adjusted according to the co-culture system (such as types and proportions of organoids, blood cells, and drugs, and the composition of the medium), the evaluation technique adopted in the toxicity evaluating step, or the attributes of the patient from which the cells used for fabricating an organoid are derived (e.g., drug toxicity-related genes). Further, the amount of the drug to be added in the evaluation method using the "co-culture system of an organoid and blood cells" in the present invention can be adjusted with reference to the amount of the drug to be added in the evaluation method using a culture system for conventional various cells, organoids, or the like (e.g., the numerical range including the upper and lower limits can be changed, as required, for improving the evaluation accuracy, or the like, by the present invention). For example, in the case of evaluating hepatotoxicity using a liver organoid, ampicillin may be added at concentrations of 20 mg/mL or less, (e.g., a number of concentrations included in the range of 0 to 20 mg/mL, and the same can be applied to the numerical ranges of other drugs), amoxicillin may be added at a concentration of 1.0 mg/mL or less, cephalexin may be added at a concentration of 2.0 mg/mL or less, and levofloxacin may be added at a concentration of 4.0 mg/mL or less, to a medium for liver organoids.

### <Toxicity evaluating step>

The toxicity evaluating step that is the second step in the method for evaluating drug toxicity of the present invention is a step of evaluating drug toxicity to an organoid.

An evaluation technique or criteria in the toxicity evaluating step can be appropriately adopted according to the types of the organoid, blood cells, and drug used, and the type of drug toxicity, etc. The type of drug toxicity refers to hepatocellular injury type, mixed type, cholestasis type, fulminant hepatitis, or the like, for example, in the case of hepatotoxicity.

In one embodiment of the present invention, the presence or absence of drug toxicity or the intensity of drug toxicity can be evaluated in the toxicity evaluation by detecting dead cells from the cells that have undergone the drug adding step using a reagent (e.g., propidium iodide ;PI), determining the proportion of dead cells in the cells (cell death percentage), and comparing it with the control. The cell death due to the drug adding step reflects hepatotoxicity of the hepatocellular injury type. For example, the drug tested can be evaluated as being less likely to have drug toxicity to the patient (low toxicity) when the cell death percentage is a predetermined criterion or less. Alternatively, for example, the drug tested can be evaluated as being less likely to have drug toxicity to a patient (low toxic) when the results of the cell death percentage for the drug tested are compared between the case of using an organoid derived from a patient having developed drug toxicity and the case of using an organoid derived from a subject who has been confirmed not to develop drug toxicity (healthy person), and there is no statistically significant difference, preferably both cell death percentages are a predetermined criterion or less. The cell death percentage may be replaced, for example, with the " increased ratio of cell death " converted as a ratio to the cell death percentage in the control without addition of the drug to the medium as the reference.

### -Method for presuming biomarker-

The method for presuming a biomarker of the present invention comprises a step of performing the method for evaluating drug toxicity of the present invention (such as the drug adding step and the toxicity evaluating step) for multiple specimens of the organoid and comparing substances (e.g., cytokine) in the culture supernatants between specimens with drug toxicity (specimens that have an index of drug toxicity higher than a predetermined criterion and are considered to have drug toxicity) and specimens without drug toxicity (specimens that have an index of drug toxicity lower than the predetermined criterion and are not considered to have drug toxicity), to presume a substance (e.g., cytokine) to serve as a biomarker.

In one embodiment of the present invention, the substance in the culture supernatants to be compared, that is, the substance to serve as a biomarker is cytokine. Involvement of the immune system (e.g., release of inflammatory cytokines from T cells) may contribute to the mechanism of action of drug toxicity (e.g., liver injury). However, it is not clarified whether or not the immune system is involved in all drugs, and there are some drugs in which the immune system is unknown to be involved but it is unclear whether or not the immune system is not involved actually. Further, causes other than the immune system may be possibly involved in the mechanism of action. Application of the method for evaluating drug toxicity of the present invention enables involvement of the immune system in drug toxicity and the other causes to be analyzed.

For example, it is considered that a culture supernatant of a specimen in which cytotoxicity is caused by a tested drug like an organoid derived from a patient contains cytokines (such as inflammatory cytokines) released from the organoid in a certain concentration. Accordingly, the concentrations of various cytokines in the culture supernatant are compared between specimens with cytotoxicity (such as an organoid derived from a patient) and specimens without cytotoxicity (such as an organoid derived from a healthy individual), so that the presence or absence of cytokines with significantly higher concentrations than the latter can be detected in the former specimens. Cytokines with significantly higher concentrations in the culture supernatant of specimens with cytotoxicity can be presumed as biomarkers of drug toxicity.

Such a method for presuming a biomarker can be applied to substances other than the cytokines, such as proteins other than the cytokines, nucleic acid molecules such as miRNA or exosomes containing them, metabolites (lipid mediators, particularly lipoxin derived from arachidonic acid, resolvin/protectin derived from ω3 fatty acid (DHA/EPA), or the like), as long as they may be biomarkers that may be possibly contained in the culture supernatant. Causes other than the immune system may possibly are involved in the mechanism of action of drug toxicity, and substances in the culture supernatant to be compared can be selected depending on the purpose of analysis.

### -Method for screening a drug-

The method for screening a drug of the present invention comprises a step of performing the method for evaluating drug toxicity of the present invention (such as the drug adding step and the toxicity evaluating step) for multiple drugs using an organoid derived from a patient having developed drug toxicity, to select a drug with low drug toxicity.

More specifically, the "drug with low drug toxicity" refers to a drug evaluated as having no drug toxicity such as having a toxicity (index) of a predetermined criterion or less or having no significant difference from the subject, in the toxicity evaluating step of the method for evaluating drug toxicity.

The method for screening a drug of the present invention can be utilized, for example, for determining drugs that can be administered to a patient having developed drug toxicity (which may be commercially available pharmaceutical products or may be drugs in the test stage) and achieving both improvement of the therapeutic effect and reduction of the risk. For example, multiple drugs as candidates for administration to a patient from which an organoid is derived are selected from drugs (such as antibacterial drugs) that possibly develop drug toxicity (such as hepatotoxicity), and the method for evaluating drug toxicity of the present invention is performed on the drugs selected, so that drugs with low drug toxicity, that is, drugs that can be administered to the patient can be selected.

Further, the method for screening a drug of the present invention can be also used for selecting drugs with lower toxicity which are less likely to cause cytotoxicity to patients with various attributes by preparing multiple organoids derived from patients who have developed drug toxicity (e.g., those with hepatotoxicity-related genes different from each other) and integrating the results of selecting drugs with low drug toxicity for each organoid.

Further, the organoid may be an organoid derived from a subject who is unknown whether or not to develop drug toxicity and can be used to confirm drugs with low drug toxicity when administered to the subject so as to select an appropriate drug from the drugs confirmed.

### -Kit-

The kit of the present invention comprises at least an anorganoid and blood cells. Such a kit can be used for performing the method for evaluating drug toxicity of the present invention and the like. The technical matter of the organoid and the blood cells used in the kit is as described for the method for evaluating drug toxicity or the like in this description.

The kit of the present invention comprises at least an organoid derived from a patient with drug toxicity as an organoid and may further contain one derived from a healthy individual (subject who does not develop drug toxicity) as a subject. Further, the organoid derived from a patient with drug toxicity may be one type, or may be two or more types (e.g., those with drug toxicity-related genes different from each other).

The kit of the present invention preferably further comprises a dead cell detection reagent. Examples of the dead cell detection reagent include propidium iodide (PI). Depending on the application of the kit, particularly, depending on the evaluation technique adopted in the toxicity evaluating step of the method for evaluating drug toxicity of the present invention or the technique adopted in the method for presuming a biomarker or the method for screening a drug of the present invention, another reagent can be used instead of or in addition to the reagent for detecting cells.

### -Culture-

The culture of the present invention comprises an organoid, blood cells, and a drug. Such a culture corresponds to a culture prepared by the drug adding step and subjected to the toxicity evaluating step, in the method for evaluating drug toxicity of the present invention. The culture may be contained in a culture vessel such as a dish (Petri dish) or may be contained in multiple wells formed on a plate (content in each well or an assembly of such contents).

### Examples

### [Example 1] Liver organoid toxicity test with antibacterial drug

### [Experimental method]

### (1) Fabrication of human vascular endothelial cells (ECs)

Each of iPS cells derived from human healthy person (1383D2; Kyoto University iPS Research Institute) and iPS cells derived from patients with drug-induced liver injury (16-24; Yokohama City University) were cultured at 5% CO₂ and 37°C for 3 days in a medium obtained by adding 1% B-27 Supplements (GIBCO), BMP4 (25 ng/ml) and CHIR99021 (8 µM) to DMEM/F-12 (Gibco) (10 ml), to induce mesodermal cells. The mesodermal progenitor cells obtained were further cultured at 5% CO₂ and 37°C for 7 days in a medium obtained by adding VEGF (200 ng/ml) and Folskolin (2 µM) to Stempro-34 SFM (Gibco) (10 ml), to obtain CD31-positive, CD73-positive and CD144-positive human vascular endothelial cell populations.

### (2) Fabrication of human hepatic endoderm cells (HEs)

Each of iPS cells derived from human healthy person (1383D2; Kyoto University iPS Research Institute) and iPS cells derived from patients with drug-induced liver injury (16-24; Yokohama City University) were cultured at 5% CO₂ and 37°C for 5 days in a medium obtained by adding Wnt3a (50 ng/mL) and activin A (100 ng/ml) to a basal medium RPMI (FUJIFILM Corporation) (2 ml), to induce endodermal progenitor cells. The endodermal progenitor cells obtained were further cultured at 5% CO₂ and 37°C for 5 days in a medium obtained by adding 1% B27 Supplements (GIBCO) and FGF2 (10 ng/ml) to the basal medium, to obtain AFP-, ALB-, and HNF4α-positive human hepatic endoderm cell populations.

### (3) Fabrication of human mesenchymal cells (MCs)

Each of iPS cells derived from human healthy person (1383D2; Kyoto University iPS Research Institute) and iPS cells derived from patients with drug-induced liver injury (16-24; Yokohama City University) were cultured at 5% CO₂ and 37°C for 3 days in a medium obtained by adding 1% B-27 Supplements (GIBCO), BMP4 (25 ng/ml) and CHIR99021(8 µM) to a basal mixed medium DMEM/F-12 (Gibco) (10 ml), to induce mesodermal progenitor cells. The mesodermal progenitor cells obtained were further cultured at 5% CO₂ and 37°C for 3 days in the same medium to which PDGFBB and activin A were added. The cultured cells were collected, re-seeded on a new gelatin-coated plate, and further cultured at 5% CO₂ and 37°C for 4 days in a medium obtained by adding bFGF and BMP4 to the basal mixed medium, to obtain FOXF1-, COL4A-, ALCAM-, and CD73-positive human mesenchymal cell populations.

### (4) Fabrication of organoid (three-dimensional structure)

The human hepatic endoderm cells (HEs), human vascular endothelial cells (ECs) and human mesenchymal cells (MCs) fabricated above were mixed at a cell number ratio of 10:7:1 (total number of 18 × 10⁵) and the mixture was co-cultured at 5% CO₂ and 37°C in a three-dimensional culture vessel, Elplasia (available from KURARAY CO., LTD.) for one day, to fabricate an aggregate. In this co-culture, 2 ml of a medium (referred to as "medium for organoids" in this description) in which a medium for hepatocytes (A) obtained by adding FBS (5%), HGF (10 ng/ml), OSM (20 ng/ml), and Dex (100 nM) to HCM (Lonza), and a medium for vascular endothelial cells (A) obtained by adding VEGF (50 ng/ml) and FGF2 (10 ng/ml) to Stempro-34 SFM (Gibco) were mixed at a volume ratio of 1:1 was used as a culture medium.

### (5) Co-culture of organoid and peripheral blood mononuclear cells (PBMCs) and addition of antibacterial drug

The organoid fabricated was further cultured at 5% CO₂ and 37°C for 114 to 15 days in the medium for organoids. The organoid cultured was collected and seeded in a 96-well U-bottom low-adsorption culture vessel (such as Corning, Sumitomo Bakelite Co., Ltd.) at 50 to 70 per well. PBMCs (HEMs) derived from multiple persons and cryopreserved in a liquid nitrogen tank were thawed and suspended in organoid mediums to which antibacterial drugs (ampicillin sodium, amoxicillin trihydrate, cephalexin, and levofloxacin hemihydrate) were each added in an appropriate concentration. Each PBMC suspension obtained was added to the wells with the organoid seeded to 2 × 10⁵ PBMCs per well and mixed.

### (6) Toxicity test for antibacterial drugs using reagent for detecting cell death (propidium iodide)

An organoid and PBMCs were co-cultured at 5% CO₂ and 37°C for 24 hours in a medium containing an antibacterial drug, and then propidium iodide (PI, available from DOJINDO LABORATORIES) was added to the medium, followed by reaction at 5% CO₂ and 37°C for 30 minutes. After the completion of the reaction, 100 µL of the culture supernatant was removed, and 100 µL of PBS(-) was added thereto, followed by standing for 1 minute to precipitate only the organoid, and then 100 µL of the supernatant was removed again. This step was repeated twice, to remove the PBMCs and wash the PI solution. 100 µL of PBS(-) was added thereto again, and thereafter a bright field image and a fluorescence image (excitation wavelength: 530 nm and fluorescence wavelength: 620 nm) were obtained using a fluorescence microscope (such as KEYENCE BZ-X Series). The fluorescence intensity and the organoid area in each image obtained were quantified using an image analysis software, Fiji, to calculate the PI staining rate per organoid area (PI staining rate = PI fluorescence intensity/organoid area). The magnification of the PI staining rate using a control with no drug added was calculated to quantify the increased ratio of dead cells.

### [Results]

(1) In this test system containing PBMCs, ampicillin significantly increased cell death in the liver organoids derived from strains of patients with drug-induced liver injury (16-24) in a concentration-dependent manner (Table 1).

**[Table 1]**

| | PBMC | Concentration of drug (Ampicillin Na) (mg/ml) | Increased ratio of dead cells (to: no drug added =1) (average ± SE) |
|---|---|---|---|
| DILI patient-derived organoid | Present | 16.6 | 4.43 ± 0.88* |
| | Absent | 16.6 | 1.36 + 0.61 |
| Healthy person-derived organoid | Present | 16.6 | 0.68 ± 0.17 |
| | Absent | 16.6 | 0.47 ± 0.09 |

| | | | |
|---|---|---|---|
| * p < 0.05 | | | |

### (2) As a result of toxicity tests using multiple antibacterial drugs, the drugs were classified into toxic compounds (ampicillin and levofloxacin hemihydrate) and nontoxic compounds (amoxicillin trihydrate and cephalexin) (Table 2).

**[Table 2]**

| | PBMC | Concentration of drug (Amoxicillin trihydrate) (mg/ml) | Increased ratio of dead cells (to: no drug added =1) (average ± SE) |
|---|---|---|---|
| DILI patient-derived organoid | Present | 0.83 | 0.81 ± 0.15 |
| | Absent | 0.83 | 1.66 + 0.24 |
| Healthy person-derived organoid | Present | 0.83 | 2.28 ± 0.71 |
| | Absent | 0.83 | 1.45 ± 0.04 |
| | | | |

| | PBMC | Concentration of drug (Cephalexin) (mg/ml) | Increased ratio of dead cells (to: no drug added =1) (average ± SE) |
|---|---|---|---|
| DILI patient-derived organoid | Present | 1.67 | 0.83 ± 0.19 |
| | Absent | 1.67 | 2.16 ± 0.35 |
| Healthy person-derived organoid | Present | 1.67 | 1.34 ± 0.41 |
| | Absent | 1.67 | 1.44 ± 0.27 |
| | | | |

| | PBMC | Concentration of drug (Levofloxacin hemihydrate) (mg/ml) | Increased ratio of dead cells (to: no drug added =1) (average ± SE) |
|---|---|---|---|
| DILI patient-derived organoid | Present | 3.33 | 3.64 ± 0.25* |
| | Absent | 3.33 | 11.06 ± 0.43* |
| Healthy person-derived organoid | Present | 3.33 | 3.19 ± 0.93 |
| | Absent | 3.33 | 4.29 ± 1.45 |

| | | | |
|---|---|---|---|
| * p < 0.05 | | | |

## Claims

1. A method for evaluating drug toxicity, comprising:
a step of adding a drug to a co-culture system of an organoid and blood cells; and
a step of evaluating the toxicity of the drug to the organoid.

2. The method for evaluating drug toxicity according to claim 1, wherein the blood cells are immunocompetent cells.

3. A method for presuming a biomarker, comprising a step of performing the method for evaluating drug toxicity according to claim 1 or 2 for multiple specimens of the organoid and comparing substances in culture supernatants between specimens having drug toxicity and specimens without drug toxicity to presume a substance to serve as a biomarker.

4. A method for screening a drug, comprising a step of performing the method for evaluating drug toxicity according to claim 1 or 2 for multiple drugs using an organoid derived from a patient having developed drug toxicity to select a drug with low drug toxicity.

5. A kit for evaluating drug toxicity, comprising an organoid and blood cells.

6. The kit according to claim 5, further comprising a dead cell detection reagent.

7. A culture comprising an organoid, blood cells and a drug.
